(19) Europäisches Patentamt
European Patent Office
Office européen des brevets

(11) **EP 3 373 904 B1**

(12) **EUROPEAN PATENT SPECIFICATION**

(45) Date of publication and mention
of the grant of the patent:
**24.03.2021 Bulletin 2021/12**

(51) Int Cl.:
*A61K 8/81* (2006.01)     *A61K 8/22* (2006.01)
*A61K 8/891* (2006.01)     *A61Q 11/02* (2006.01)

(21) Application number: **16820537.5**

(22) Date of filing: **16.12.2016**

(86) International application number:
**PCT/US2016/067076**

(87) International publication number:
**WO 2017/106588 (22.06.2017 Gazette 2017/25)**

(54) **TOOTH WHITENING COMPOSITIONS COMPRISING PEROXIDE COMPLEX AND PERCARBONATE SALT**

ZAHNBLEICHZUSAMMENSETZUNGEN MIT PEROXIDKOMPLEX UND PERCARBONATSALZ

COMPOSITIONS DE BLANCHIMENT DES DENTS COMPRENANT UN COMPLEXE DE PEROXYDE ET DU SEL DE PERCARBONATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2015 US 201514974723**

(43) Date of publication of application:
**19.09.2018 Bulletin 2018/38**

(73) Proprietor: **Colgate-Palmolive Company**
**New York, NY 10022 (US)**

(72) Inventors:
• **EVANS, Lauren**
**Highland Park**
**NJ 08904 (US)**

• **HASSAN, Mahmoud**
**Somerset**
**New Jersey 08873 (US)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
**US-A1- 2005 036 956**     **US-A1- 2006 045 854**
**US-A1- 2015 305 843**

## Description

### BACKGROUND

In a mammal, a tooth is comprised of an inner dentin layer and an outer hard enamel layer that is the protective layer of the tooth. The enamel layer of a tooth is naturally an opaque white or slightly off-white color. It is the enamel layer that can become stained or discolored. The enamel layer of a tooth is composed of hydroxyapatite mineral crystals that create a somewhat porous surface. These hydroxyapatite crystals form microscopic hexagonal rods or prisms that make up the enamel surface. As a result, the surface of the enamel layer presents microscopic spaces or pores between the prisms. It is believed that this porous nature of the enamel layer is what allows staining agents and discoloring substances to permeate the enamel and discolor the tooth. These remaining substances can occupy the microscopic spaces and eventually alter the color of the tooth.

Many substances that a person confronts or comes in contact with on a daily basis can stain or reduce the whiteness of one's teeth. In particular, the foods, tobacco products, and fluids that one consumes tend to stain one's teeth. These products or substances tend to accumulate on the enamel layer of the tooth and form a pellicle film over the teeth.

These staining and discoloring substances can then permeate the enamel layer. This problem occurs gradually over many years, but imparts a noticeable discoloration of the enamel of one's teeth. So long as the discolored teeth are still healthy and do not pose any health risk or problem, a product or substance that would whiten the discolored teeth would be advantageous. Products and substances that are presently available to whiten teeth may include a variety of different ingredients, but the primary active ingredient is a peroxide agent formulated into a liquid, paste or gel carrier.

It is important that a tooth whitening product that is to be used at home or in private by the consumer be safe and easy to use and be stable and retain its whitening efficacy during its storage on retail store shelves as well as over the period of use by the consumer.

There is a need in the art for improved tooth whitening compositions that can provide good peroxide retention on the tooth surface, enhanced whitening efficacy, and a stable formulation.

US 2005/036956 A1 discloses non-aqueous tooth whitening compositions.

US 2006/045854 A1 describes oral care compositions for whitening teeth.

US 2015/0305843 A1 discloses a regimen for whitening teeth comprising administering a bleaching agent to tooth.

### BRIEF SUMMARY

Disclosed herein are tooth whitening compositions comprising: (a) a peroxide complex comprising hydrogen peroxide and an N-vinyl heterocyclic polymer; (b) from 0.1% to 6%, by weight, sodium percarbonate; and (c) at least one hydrophobic polymer carrier, wherein the hydrogen peroxide of the peroxide complex is present in the tooth whitening composition in an amount ranging from 3% to 5% by weight, such as about 4.5% by weight.

In certain exemplary embodiments of the tooth whitening compositions disclosed herein, the sodium percarbonate is present in an amount of about 2% by weight. In certain embodiments, the at least one hydrophobic carrier is chosen from polysiloxane polymers, such as, for example, dimethicone, and in certain exemplary embodiments, the tooth whitening compositions disclosed herein have a viscosity ranging from 10,000 cps to 900,000 cps, measured on a Brookfield Viscometer at 25 °C.

Further disclosed herein are methods of whitening the surface of a tooth comprising: contacting the surface of the tooth with a tooth whitening composition for a duration of time sufficient to effect whitening of the surface of the tooth, wherein the tooth whitening composition comprises a peroxide complex comprising hydrogen peroxide and an N-vinyl heterocyclic polymer; from 0.1% to 6%, by weight, sodium percarbonate; and at least one hydrophobic polymer carrier, and wherein the hydrogen peroxide of the peroxide complex is present in the tooth whitening composition in an amount ranging from 3% to 5% by weight, such as about 4.5% by weight. In certain embodiments, the duration of time ranges from 1 minute to 45 minutes.

In certain embodiments of the tooth whitening methods disclosed herein, the sodium percarbonate is present in the tooth whitening composition in an amount of about 2% by weight. In other exemplary embodiments of the tooth whitening methods disclosed herein, the hydrophobic polymer carrier of the tooth whitening composition is chosen from polysiloxane polymers, such as, for example, dimethicone, and in certain exemplary embodiments, the tooth whitening composition has a viscosity ranging from 10,000 cps to 900,000 cps, measured on a Brookfield Viscometer at 25 °C.

Further disclosed herein are methods for making a tooth whitening composition comprising adding a peroxide complex comprising hydrogen peroxide and an N-vinyl heterocyclic polymer and from 0.1% to 6% by weight, sodium percarbonate to at least one hydrophobic polymer carrier; and mixing the peroxide complex, the sodium percarbonate, and the at least one hydrophobic polymer carrier to form a homogeneous dispersion, wherein the hydrogen peroxide is present in the tooth whitening composition in an amount ranging from 3% to 5% by weight, such as about 4.5% by weight. In certain exemplary embodiments of the methods for making the tooth whitening compositions disclosed herein, the sodium percarbonate is present in an amount of about 2% by weight, and in certain embodiments, the methods further comprise adding at least one flavoring agent. In other exemplary embodiments of the methods for making the tooth whitening compositions disclosed herein, the hydrophobic polymer carrier of the tooth whitening composition is chosen from polysiloxane polymers, such as, for example, dimethicone, and in certain exemplary embodiments, the tooth whitening composition has a viscosity ranging from 10,000 cps to 900,000 cps, measured on a Brookfield Viscometer at 25 °C.

Further areas of applicability of the present invention will become apparent from the detailed description provided hereinafter. It should be understood that the detailed description and specific examples, while indicating embodiments of the invention, are intended for purposes of illustration only and are not intended to limit the scope of the invention.

## DETAILED DESCRIPTION

The following description of the preferred embodiments is merely exemplary in nature and is in no way intended to limit the invention, its application, or uses.

As used throughout, ranges are used as shorthand for describing each and every value that is within the range. Any value within the range can be selected as the terminus of the range.

As used herein, the term "one or more of" with respect to a listing of items such as, for example, A and B, means A alone, B alone, or A and B. The term "at least one of" is used to mean one or more of the listed items can be selected.

Unless otherwise specified, all percentages and amounts expressed herein and elsewhere in the specification should be understood to refer to percentages by weight. The amounts given are based on the active weight of the material.

Disclosed herein are stable tooth whitening compositions comprising a peroxide complex and sodium percarbonate. The compositions disclosed herein comprise a liquid suspension of peroxide whitening agents in a liquid hydrophobic polymer, wherein the suspension, when applied to the teeth, is sufficiently viscous to form an adherent, continuous layer of the peroxide-containing composition on dental enamel surfaces.

The liquid hydrophobic polymer provides a stable vehicle that prevents decomposition of the peroxide whitening agents during storage and before use. Once applied to a tooth surface, the saliva on the tooth enamel surface to which the composition is applied will either dissolve or disintegrate the peroxide-containing layer, resulting in a rapid decomposition of the peroxide, which in turn provides the whitening effect.

It has been discovered that the addition of a sodium percarbonate to a tooth whitening composition comprising hydrogen peroxide, such as hydrogen peroxide in a peroxide complex, dramatically enhances the whitening effect of the composition. While not wishing to be bound by theory, it is believed that the sodium percarbonate may help to break the hydrophobic matrix as a result of the micro bubbles generated when the percarbonate comes into contact with water, such as saliva. This disruption is believed to enhance the release of the whitening agents, such as the hydrogen peroxide. Additionally, the percarbonate provides an alkaline medium. It is known that hydrogen peroxide functions better in an alkaline environment than in acidic or neutral pH conditions in terms of whitening efficacy. Therefore, it is believed that, surprisingly and unexpectedly, the sodium percarbonate provides a synergistic whitening effect when combined with the hydrogen peroxide of the peroxide complex in the tooth whitening compositions disclosed herein.

In some embodiments, the tooth whitening composition disclosed herein is a viscous liquid, such as a gel, which maintains its consistency during storage, enabling the product to be painted on the tooth surface with a soft applicator pen or brush. In some embodiments, the composition disclosed herein provides a stable vehicle that prevents the decomposition of the hydrogen peroxide and/or the sodium percarbonate during storage and before use. As discussed above, it is believed that the sodium percarbonate enhances the breakdown of the peroxide complex matrix, thereby improving the whitening efficacy of the hydrogen peroxide and the overall performance of the whitening composition.

In certain embodiments, upon application to the teeth, the applied tooth whitening composition forms an adherent layer of peroxide-containing product that has the capacity to release the peroxide whitening agent over an extended period of time, such as from about 5 minutes to about 12 hours. The applied layer adheres to the tooth surface whereby the released peroxide source or sources then whiten the teeth to which the composition is applied.

In some embodiments, the tooth whitening compositions disclosed herein are substantially anhydrous, that is, no water is added. The composition may contain trace levels of water from ingredients or from product manufacture; however, such trace levels are insubstantial and do not interfere with the hydrophobic character of the composition.

## Peroxide Complex

Disclosed herein are tooth whitening compositions comprising a peroxide complex. The peroxide complex comprises a peroxide component and a porous cross-linked polymer. As referred to herein, a "peroxide component" is any oxidizing compound comprising a bivalent oxygen-oxygen group. Peroxide components include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as percarbonate and perborate salts of alkali and alkaline earth metals such as lithium, potassium, sodium, magnesium, calcium and barium, and mixtures thereof. In various embodiments, the peroxide component comprises hydrogen peroxide, urea peroxide, sodium percarbonate and mixtures thereof. In one embodiment, the peroxide component comprises hydrogen peroxide. In one embodiment, the peroxide component consists essentially of hydrogen peroxide.

The peroxide complex also comprises an N-vinyl heterocyclic polymer. In certain embodiments, the polymer is crosslinked and adsorbs, absorbs, complexes, or otherwise retains the peroxide component. The chemical and physical characteristics of the particulate hinder the release of the peroxide compound from the polymer particulates, and in some embodiments provides controlled release of the peroxide compound. The peroxide complex may, in certain embodiments, comprise a peroxide component at a level ranging from about 0.1% to about 25%, optionally from about 1% to about 25%, optionally from about 5% to about 18% by weight of the peroxide complex. In one embodiment, the composition comprises a commercially available complex of peroxide adsorbed to cross-linked polyvinylpyrrolidone. Such products include, for example, PEROXYDONE XL-10 and PEROXYDONE K-30, marketed by ISP Corporation, Wayne, N.J., USA.

The N-vinyl heterocyclic polymer is derived from a N-heterocyclic vinyl monomer, which may comprise N-vinyl heterocyclic monomers having from 3 to 7 atoms in a heterocyclic ring, including a carbonyl carbon atom and a nitrogen heteroatom containing a vinyl group. In certain embodiments the ring contains 5 or 6 atoms, comprises heteroatoms such as sulfur or oxygen, and may be substituted or unsubstituted.

Suitable N-vinyl lactams may include: N-vinyl piperidone; N-vinyl caprolactam; N-vinyl-3-methylpyrrolidinone or piperidone, or caprolactam; N-vinyl-4-methyl pyrrolidinone, or piperidone or caprolactam; N-vinyl-5-methylpyrrolidinone or piperidone; N-vinyl-3-ethyl pyrrolidinone; N-vinyl-4,5-dimethyl pyrrolidinone; N-vinyl-5,5-dimethyl pyrrolidinone; N-vinyl-3,3,5-trimethyl pyrrolidinone; N-vinyl-5-methyl-5-ethyl pyrrolidinone; N-vinyl-3,4,5-trimethyl-3-ethyl pyrrolidinone; N-vinyl-6-methyl-2-piperidone; N-vinyl-6-ethyl-2-piperidone; N-vinyl-3,5-dimethyl-2-piperidone; N-vinyl-4,4-dimethyl-2-piperidone; N-vinyl-7-methyl caprolactam; N-vinyl-7-ethyl caprolactam; N-vinyl-3,5-dimethyl caprolactam; N-vinyl-4,6-dimethyl caprolactam; and N-vinyl-3,5,7-trimethyl caprolactam.

Embodiments containing poly-N-vinyl polylactams may include but are not limited to poly-N-vinyl pyrrolidone, poly-N-vinyl-2-piperidone, poly-N-vinyl-2-caprolactam, poly-N-vinyl-3-methyl-2-caprolacam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, poly-N-vinyl-4,5-dimethyl-2-pyrrolidone, and mixtures thereof. In certain embodiments, the polymer is selected from the group consisting of poly-N-vinyl poly-2-pyrrolidone, poly-N-vinyl-poly-2-piperidone, poly-N-vinyl-poly-2-caprolactam and mixtures thereof.

In one exemplary embodiment, the polymer is poly-N-vinyl-poly-2-pyrrolidone. The poly-N-vinyl-poly-2-pyrrolidone is also commonly known as polyvinylpyrrolidone or "PVP". PVP refers to a polymer containing vinylpyrrolidone (also referred to as N-vinylpyrrolidone, N-vinyl-2-pyrrolidone and N-vinyl-2-pyrrolidinone) as a monomeric unit. The monomeric unit consists of a polar imide group, four non-polar methylene groups and a non-polar methane group. The polymers include soluble and insoluble homopolymeric PVPs. Copolymers containing PVP include vinylpyrrolidone/vinyl acetate (also known as Copolyvidone, Copolyvidonum or VP-VAc) and vinyl pyrrolidone/dimethylamino-ethylmethacrylate.

Soluble PVP polymers among those useful herein are known in the art, including Povidone, Polyvidone, Polyvidonum, poly(N-vinyl-2-pyrrolidinone), poly (N-vinylbutyrolactam), poly(1-vinyl-2-pyrrolidone) and poly[1-(2-oxo-1 pyrrolidinyl)ethylene]. These PVP polymers are not substantially cross-linked.

In various embodiments disclosed herein, an insoluble cross-linked homopolymer may be used. Such polymers include those commonly referred to in the art as polyvinylpolypyrrolidone, cross-povidone, and cPVP, and are referred to herein as "cPVP." The homopolymer may be prepared by free radical polymerization of the monomer vinylpyrollidone.

In one embodiment, the poly-N-vinyl-poly-2-pyrrolidone has a lactam of the pyrrolidone ring that provides the hydrophilic characteristics. Without limiting the compositions, mechanisms, or functions of the compositions disclosed herein, it is believed that such groups allow the peroxide compound to bind to the cPVP. The hydrophobic characteristics attributed to the methylene groups in the ring and the linear aliphatic backbone prevent the peroxide complex from reacting with saliva while still maintaining the peroxide's availability to whiten the teeth. The surface characteristic of the cPVP may serve as a barrier to the passage of the peroxide component and may prevent the premature distribution of the peroxide component upon application of the tooth whitening composition to the oral cavity. The cPVP-linked peroxide of the peroxide complex may be released over a period of time through diffusion, temperature variance, moisture levels and other factors.

Polymer particulates useful herein may be made by well-established processes. For example, the poly-N-vinyl polylactams may be produced by polymerizing a vinyl lactam in the presence of an alkaline catalyst. Embodiments containing poly-N-vinyl polyimides may be produced by heating an N-vinyl imide in the presence of a catalyst. In alternative embodiments comprising a co-polymer of the N-vinyl heterocyclic compound, the compound may be produced by polymerizing N-vinyl heterocyclic and dissimilar vinyl monomers.

Porous cross-linked polymers among those useful herein may include those commercially available as: KOLLIDON and LUVICROSS, marketed by BASF, Mount Olive, N.J., USA; PVP K-Series or Povidone K-30 marketed by AAA International Corp., Downers Grove, Il1., USA; PVP K-30 USP24 and industry grade, PVP VA-64, PVP K-17 and PVP K-90, marketed by Peakchem, Hangzhou, China; and POLYTLASDONE INF-10, marketed by ISP Corporation, Wayne, N.J., USA. It is understood that embodiments disclosed herein are not limited to a PVP of a specific molecular weight and that any equivalent PVP of acceptable purity, such as pharmaceutical grade, is within the scope of embodiments disclosed herein.

In various embodiments, the peroxide complex is made by suspending the polymer (such as cPVP) in a suitable anhydrous organic solvent. An anhydrous solution of the peroxide component is made, such as by utilizing the same organic solvent as the PVP suspension. The peroxide solution is combined with the PVP suspension in an amount corresponding to the desired molar ratio of polymer peroxide of the peroxide complex. In one embodiment, the peroxide complex has an equal (1:1) molar ratio of hydrogen peroxide to the polymer.

Both PVP-$H_2O_2$ and cPVP-$H_2O_2$ are considered to be stable in an anhydrous environment. Upon exposure to highly aqueous environments, such as in the oral cavity, the peroxide complex, such as PVP-$H_2O_2$ or cPVP-$H_2O_2$, dissociates into individual species (PVP polymer and $H_2O_2$). In one embodiment, the PVP-$H_2O_2$ complex is about 80% by weight polyvinylpyrrolidone and about 20% by weight $H_2O_2$. In another exemplary embodiment, the cPVP-$H_2O_2$ complex is about 80% by weight polyvinylpolypyrrolidone and about 20% by weight $H_2O_2$.

In certain embodiments disclosed herein, the peroxide complex may be present in the tooth whitening composition in an amount ranging from about 0.5% to about 40%, such as from about 20% to about 30%. In certain exemplary embodiments, the amount of the peroxide complex is such that the peroxide component of the peroxide complex is present in an amount ranging from about 0.1% to about 10% of the total composition weight, such as from about 1% to about 6%, about 2% to about 4.5%, or about 4.5% of the total composition weight.

In alternate embodiments disclosed herein, the at least one peroxide compound comprises a liquid peroxide solution. The hydrophobic polymer carrier of the peroxide compound provides sufficient stability to permit the use of a liquid hydrogen peroxide. The liquid hydrogen peroxide comprises $H_2O_2$ generally contained in an aqueous water-based solution. In some embodiments, the liquid hydrogen peroxide has a concentration of peroxide to the total solution ranging from about 0.035% to about 17.5%, such as from about 3% to about 1 0% by weight, which for example may be achieved by adding a 35 wt % aqueous $H_2O_2$ solution at a concentration of from about 0.1wt% to about 50 wt%, such as about 8 wt% to about 29 wt%, or from about 15 wt% to about 25 wt%. Additionally, at least one stabilizer may be present. For example, a 3% hydrogen peroxide solution with about 0.1% to about 0.5% of at least one stabilizer may be used. Acetanilide or a similar organic material can also be used with a pyrophosphate stabilizer such as sodium acid pyrophosphate, present in an amount ranging from about 0.1% to about 1.0%, such as about 0.5%.

**Sodium Percarbonate**

Sodium percarbonate comprises sodium carbonate and hydrogen peroxide and has the chemical formula $2Na_2CO_3 \cdot 3H_2O_2$. Sodium percarbonate is an oxidizing agent, which, when dissolved in water, may yield a mixture of its sodium carbonate and hydrogen peroxide constituents.

In embodiments disclosed herein, the sodium percarbonate is present in the tooth whitening compositions in an amount ranging from 0.1% to 6%, such as from about 1% to about 2%, about 1% or about 2%.

In certain embodiments, the tooth whitening composition may further comprise at least one agent to enhance release of the peroxide in the oral cavity as a part of the peroxide component whitening agent for either the sodium percarbonate or the hydrogen peroxide of the peroxide complex discussed above. POLY-PORE, which is an allyl methacrylate crosspolymer, available from Amcol health & Beauty Solutions, Inc., is an exemplary enhancing agent.

**Hydrophobic Polymer Carrier**

The tooth whitening compositions disclosed herein may comprise an orally-acceptable carrier that comprises a hydrophobic polymer. The term "hydrophobic" or "water-insoluble" as applied to polymers and as employed herein refers to an organic polymer that is substantially non-aqueous having a water solubility of less than one gram per 100 grams of water at 25 °C. In various embodiments, a hydrophobic polymer is compatible with the peroxide complex and sodium percarbonate described herein. In certain embodiments, a hydrophobic polymer is selected for the carrier to produce a tooth whitening composition having a viscosity ranging from about 1,000 cPs to about 900,000 cPs, such as from about 10,000 cPs to about 900,000 cPs or from about 10,000 cPs to about 100,000 cPs. Any such silicone polymers that are compatible with the whitening agents described herein, such as the peroxide complex and the sodium percarbonate, and which can produce a tooth whitening composition having a desired viscosity, can be used.

In some embodiments, the hydrophobic polymers suitable for use in the present invention are referred to as "siloxane" polymers, which are also generally known in the art as "silicone" polymers, such as silicone pressure sensitive adhesives (PSA). In certain embodiments disclosed herein, the hydrophobic polymers that comprise the hydrophobic material are those in which whitening agents, such as the peroxide complex and the sodium percarbonate, can be dispersed and are well known in the art. Many such silicone polymers are commercially available. In various embodiments, a preferred silicone-based hydrophobic polymer is a polyorganosiloxane, such as polydimethylsiloxane.

Exemplary polyorganosiloxanes may be produced by condensing a silicone resin and an organosiloxane, such as a polydiorganosiloxane. Such hydrophobic polymers are an elastomeric, tacky material, adhesion of which to dental enamel surfaces can be varied by altering the ratio of silicone resin to polydiorganosiloxane in the copolymer molecule. In certain embodiments, the polymers are pressure sensitive hydrophobic polymers specifically designed for pharmaceutical use and are permeable to many drug compounds and find application for the transdermal application of various compounds. In one such embodiment, the silicone polymers are the copolymer product of mixing a silanol terminated polydiorganosiloxane, such as polydimethyl siloxane, with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone hydrophobic polymers. A catalyst, for example, an alkaline material, such as ammonia, ammonium hydroxide or ammonium carbonate, can be mixed with the silanol-terminated polydiorganosiloxane and the silicone resin to promote this crosslinking reaction. By copolymerizing the silicone resin with the silanol-terminated polydiorganosiloxane, there results a polymer with self-adhering properties and the cohesive properties of a soft elastomer matrix characteristic of pressure sensitive polymers being distinguished from the hard, non-elastomeric properties of other silicone resins. In one embodiment, hydrophobic polymers used in the carrier are available from the Dow-Corning Company under the brand name BIO-PSA.

The modification of a ratio of silicone resin to polydiorganosiloxane modifies the tackiness of the hydrophilic polymer. This ratio may, for example, be in the range of 70:30 to 50:50. For example, the BIO-PSA silicone sold by Dow-Corning is available in three silicone resin to silicone polymer ratios: namely, 65/35 (low tack), 60/40 (medium tack), and 55/45 (high tack), as modifying the silicone resin to polydiorganosiloxane ratio of the PSA will modify the tackiness of the PSA. Such a polyorganosiloxane pressure sensitive adhesive is available dissolved in either ethyl acetate solvent or dimethicone. An exemplary suitable silicone PSA is Silicone Adhesive 8-7016, commercially available from Dow Corning.

In some embodiments, the siloxane polymers that can function as part of the hydrophobic component are in the form of a fluid. Polysiloxane fluids useful herein for the hydrophobic silicone material component include those with a viscosity, at 25° C, of about 1 milliPascal-sec (mPa-s) to about 1000 mPa-s, or about 2 mPa-s to about 500 mPa-s, or about 20 mPa-s to about 400 mPa-s. Polysiloxane fluids for use herein can be linear or cyclic, and can be substituted with a wide variety of substituents. In certain embodiments, substituents include methyl, ethyl and phenyl substituents. Suitable polysiloxane fluids include linear polysiloxane polymers such as dimethicone and other low viscosity analogues of the polysiloxane materials, in certain embodiments having a viscosity, at 25° C, of 200 mPa-s or less and cyclomethicone, and other cyclic siloxanes having for example a viscosity, at 25° C, of 200 mPa-s or less. Other fluids include polysiloxane polyether copolymers and hydroxy terminated polydimethyl-siloxane fluid (e.g., Dow Corning ST-DIMETHICONOL™ 40, Dow Corning SGM 36, SGM3). Commercial examples of materials that are suitable for use herein include the DC200 series fluids marketed by Dow-Corning Corporation and the AK Fluid series marketed by Wacker-Chemie GmbH, Munchen, Germany. High molecular silicone resins with a polysiloxane blend may also be used including powdered trimethylsiloxysilicate, for example, Dow Coming 593 fluid, Wacker Belsil TMS 803. Another suitable silicone fluid from Dow Corning is Q7-9210.

In certain embodiments disclosed herein, the hydrophobic polymer carrier, such as the polydiorganosiloxane, may comprises from about 10% to about 80% by weight of the tooth whitening composition, such as, for example from about 20% to about 80%, from about 30% to about 70%, from about 30% to about 50%, or about 30%.

**Adhesion Enhancing Agents**

Adhesion enhancing agents may be used in the compositions disclosed herein to enhance the adhesive properties of the anhydrous hydrophilic polymers and include inorganic materials as well as organic natural and synthetic polymers. Inorganic materials include amorphous silica compounds that may function as thickening agents, including colloidal silica compounds available under trademarks such as CAB-O-SIL fumed silica manufactured by Cabot Corporation and distributed by Lenape Chemical, bound Brook, N.J.; ZEODENT 165 from J.M. Huber Chemicals Division, Havre de Grace, Md. 21078; and SYLOX 15 also known as SYLODENT 15, available from Davison Chemical Division of W.R. Grace Corporation, Baltimore, Md. 21203.

An exemplary amorphous, fumed silica compound is obtainable in powder form from the Degussa Company under the trade designation AEROSIL. Various types of AEROSILs are available depending on the variations in the manufacturing process. AEROSIL 200 is a hydrophilic fumed silica, having a surface area of about 200 square meters per gram, an average particle size of about 10-12 nanometers and a compacted apparent density of about 50 g/l and may be used in the tooth whitening compositions disclosed herein.

An additional exemplary adhesion enhancing agent that may be mentioned is sorbitan sebacate behenate polymer. Sorbitan sebacate behenate polymer is also known as sorbitol sebacic acid copolymer behenate, and is copolymer of sorbitol and sebacic acid esterified with behenic acid. It is known for use in controlling viscosity. Additional adhesion enhancing agents may be used in accordance with various embodiments of the tooth whitening composition disclosed herein. Exemplary adhesion enhancing agents disclosed herein include inorganic materials as well as organic natural and synthetic polymers. In certain exemplary embodiments, the inorganic adhesion enhancing material, such as silica, may be surface treated to compatibilize the adhesion enhancing agent with the hydrophobic components in the tooth whitening composition.

Organic polymers useful as adhesion enhancing agents that may be mentioned include hydrophilic polymers such as Carbomers like carboxymethylene polymers such as acrylic acid polymers, and acrylic acid copolymers. Carboxypolymethylene is a slightly acidic vinyl polymer with active carboxyl groups. A carboxypolymethylene that may be used in the compositions disclosed herein is a copolymer of acrylic acid cross linked with approximately 0.75% to approximately 1.5% polyallyl sucrose and is sold under the trade designation Carbopol 934, 974 by B.F. Goodrich.

Small amounts of an anhydrous solvent such as ethanol as well as humectants such as glycerin may also be used to adjust the viscosity of the liquid compositions disclosed herein and may be present in the non-aqueous tooth whitening compositions in amounts ranging from about 0.1% to about 25% by weight, such as about 0.3% to about 20% by weight.

Hydrophobic organic materials are also useful as adhesion enhancing agents and include hydrophobic materials such as waxes such as bees wax, mineral oil, plastigel (a blend of mineral oil and polyethylene), petrolatum, white petrolatum, versagel (blend of liquid paraffin, butene/ethylene/styrene hydrogenated copolymer) acrylate and vinyl acetate polymers and copolymers, polyethylene waxes, silicone polymers such as dimethicone, silicone elastomers, organosiloxane resins, silicone gums and polyvinyl pyrrolidone/vinyl acetate copolymers.

According to certain embodiments disclosed herein, the tooth whitening composition may be anhydrous, and in certain embodiments the tooth whitening composition may be substantially free of polyethylene, for example substantially free of plastigel. As used herein, "substantially free of" means about 0% by weight or an amount of that is so low as to not have a reasonable chemical effect on the composition.

The tooth whitening compositions disclosed herein may further comprise crospovidone (poly[N-vinyl-2-pyrrolidone]) as an adhesion enhancing agent. Crospovidone may be present in the composition in an amount ranging from about 10% to about 30%, by weight relative to the total weight of the tooth whitening composition, such as ranging from about 15% to about 25%, or ranging from about 18% to about 25%.

Also effective as adhesion enhancing agents are liquid hydrophilic polymers including polyethylene glycols, nonionic polymers of ethylene oxide having the general formula: $HOCH_2(CH_2OCH_2)_nCH_2OH$, wherein n represents the average number of oxyethylene groups. Polyethylene glycols available from Dow Chemical are designated by a number such as 200, 300, 400, 600, and 2000, which represents the approximate average molecular weight of the polymer, as well as nonionic block copolymer of ethylene oxide and propylene oxide of the formulas: $HO(CH_4O)_a(C_3H_6O)_b(C_2H_4O)_cH$.

The block copolymer may be chosen (with respect to a, b and c) such that the ethylene oxide constituent comprises from about 65% to about 75% by weight, of the copolymer molecule and the copolymer has an average molecular weight of from about 2,000 to about 15,000 with the copolymer being present in the tooth whitening composition in such concentration that the composition is a gel at room temperature (about 23 °C).

One block copolymer for use herein is available commercially from BASF and designated PLURAFLO L1220, which has an average molecular weight of 9,800. The hydrophilic poly (ethylene oxide) block averages 65% by weight of the polymer.

As used herein, the term "hydrophilic" polymer refers to an organic polymer that has a water solubility of at least about one gram per 100 grams of water at 25 °C. The term "non-aqueous" as used herein means the presence of less than 10% by weight water in the composition.

At least one additional adhesion enhancing agent may be employed in compositions of various embodiments disclosed herein may present in an amount ranging from 1% to 80% by weight, relative to the total weight of the tooth whitening composition, such as, for example, ranging from about 1.5% to 75% by weight or about 25% to about 70% by weight.

**Additional Components**

Other components may further be included in the whitening compositions disclosed herein, and include surfactants, flavoring agents, sweetening agents, additional whitening agents, desensitizing agents, anti-microbial agents, anti-caries agents, anti-calculus agents, anti-inflammatory agents, vitamins, pigments and coloring agents, enzymes, preservatives, and tartar control agents, for example.

Nonionic surfactants that are compatible with peroxide compounds serve as a solubilizing, dispersing, emulsifying and wetting agents and may be effective to solubilize a flavor if included in the liquid whitening composition. An exemplary nonionic surfactant is a water soluble poly oxyethylene monoester of sorbitol with a C 1 0 to C18 fatty acid, marketed commercial under the Tween trademark. The Tween surfactants are mixtures of C10 to C18 fatty acid esters of sorbitol (and sorbitol anhydrides), consisting predominately of the monoester, condensed with about 10-30, such as about 20, moles of ethyleneoxide. The fatty acid (aliphatic hydrocarbonyl monocarboxylic acid) may be saturated or unsaturated, e.g., lauric, palmitic, stearic, oleic acids. Polysorbate 20 (e.g., Tween 20) may be used in the compositions disclosed herein and is commonly referred to as polyoxyethylene (20) sorbitan monolaurate. In certain embodiments, the nonionic surfactant may be present in the composition in an amount ranging from about 0 to about 50% by weight, such as about 0.5% to about 40% by weight of the liquid composition.

In certain embodiments disclosed herein, the tooth whitening composition may further comprise at least one flavoring agent. The at least one flavoring agent, may, for example, be selected from essential oils, as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent may be incorporated in the tooth whitening compositions disclosed herein at a concentration ranging from 0.01% to about 2% by weight, such as about 0.1% to about 0.8% by weight, about 0.6% or about 0.8% by weight.

In embodiments where the tooth whitening composition is sweetened, at least one sweetening agent may be used as an alternative or as a complement to the at least one flavoring agent. Suitable sweetening agents may be water-soluble and include, for example, sodium saccharin, sodium cyclamate, xylitol, perillartien, D-tryptophan, aspartame, dihydrochalcones and the like. The at least one sweetening agent may be present in the tooth whitening composition in an amount ranging from about 0.01% to about 1% by weight, such as about 0.3%.

In certain embodiments, peroxide decomposition activators such as sodium bicarbonate, sodium carbonate, and manganese gluconate may be incorporated in the liquid whitening gel compositions disclosed herein. The activator may be relatively nonactive with the peroxide whitening agent when present in the stored liquid composition due to the anhydrous nature of the compositions. The activator functions to react with the peroxide to release oxygen when the liquid whitening composition applied to the teeth is contacted with saliva in the oral cavity. The peroxide activator may be present in the liquid compositions disclosed herein in an amount ranging from about 0 to about 10% by weight, such as about 1% to about 5% by weight.

Exemplary antimicrobial agents may include those typically used in oral care compositions, such as Triclosan, chlorhexidine, copper-, zinc-and stannous salts such as zinc citrate, zinc sulfate, zinc glycinate, sodium zinc citrate and stannous pyrophosphate, sanguinarine extract, metronidazole, quaternary ammonium compounds, such as cetylpyridinium chloride; bisguanides, such as chlorhexidine digluconate, hexetidine, octenidine, alexidine; and halogenated bisphenolic compounds, such as 2,2'methylenebis-(4-chloro-6-bromophenol).

Exemplary anti-inflammatory agents may include those typically used in oral care compositions, such as ibuprofen, flurbiprofen, aspirin, indomethacine. Exemplary anti-caries agents may include ingredients such as sodium-, calcium-, magnesium-and stannous fluoride, aminefluorides, disodium monofluorophosphate and sodium trimetaphosphate. Exemplary vitamins may include ingredients such as Vitamin C. Exemplary desensitizing agents may include ingredients such as potassium citrate, potassium chloride, potassium tartrate, potassium bicarbonate, potassium oxalate, potassium nitrate and strontium salts. Exemplary anti-calculus agents may include ingredients such as pyrophosphate salts including the mono, di, tri and tetra alkali metal and ammonium pyrophosphate and tripolyphosphate salts. Exemplary enzymes may include papain and glucoamylase.

Some embodiments provide compositions wherein at least one of ingredients is a fluoride ion source selected from stannous fluoride, sodium fluoride, potassium fluoride, sodium monofluorophosphate, sodium fluorosilicate, ammonium fluorosilicate, amine fluoride, and ammonium fluoride.

The viscosity of the tooth whitening composition disclosed herein may be greater than about 1,000 centipoise (cPs) and less than about 900,000 cPs, such as ranging from about 10,000 cPs to about 100,000 cPs; from about 50,000 to about 900,000 cPs, or ranging from about 200,000 cPs to about 600,000 cPs.

In some embodiments, the composition is in the form of a gel.

Also disclosed herein are methods for whitening a surface of a tooth in an oral cavity of a human or other animal subject which comprises (a) applying a tooth whitening composition as disclosed herein to the tooth surface to be whitened for a plurality of minutes per day; and (b) repeating step (a) for multiple days to thereby whiten the teeth. Also disclosed herein is a method for whitening a surface of a tooth comprising contacting the surface of the tooth for a duration of time sufficient to effect whitening of the surface of the tooth with a composition comprising a peroxide complex comprising hydrogen peroxide and an N-vinyl heterocyclic polymer; sodium percarbonate; and at least one hydrophobic polymer carrier. In certain embodiments, the methods for whitening a surface of a tooth disclosed herein comprise applying to the surface of the tooth a composition comprising a peroxide complex comprising hydrogen peroxide and an N-vinyl heterocyclic polymer; sodium percarbonate; and at least one hydrophobic polymer carrier, wherein the hydrogen peroxide of the peroxide complex is present in the tooth whitening composition in an amount ranging from about 3% to about 5%, such as about 4.5%.

Exemplary methods disclosed herein comprise contacting the tooth whitening composition with the surface of the tooth. The contacting may occur for a duration of time sufficient to satisfactorily effect whitening of the teeth. Thus, the contacting occurs for a sufficient period of time to at least partially whiten teeth. This can be a period of time ranging from about 1 minute to about 2 hours or longer. In certain embodiments, the contacting is for a period of time ranging from about 1 minute to about 5 minutes, from about 1 minute to about 45 minutes, from about 5 minutes to about 45 minutes, or from about 5 minutes to about 30 minutes.

In certain embodiments disclosed herein, a tooth whitening composition may be effective over a longer period of time, since it is not significantly diluted or washed away in the oral cavity during the treatment time. The tooth whitening composition can be removed as and when required, at will, by an employment of standard oral hygiene procedures such as brushing or by rinsing with an alcoholic mouthwash. While in place, the composition may release agents contained therein at a slow, relatively constant rate and in concentration sufficient to effect stain removal from the teeth.

Whiteness may be measured by any means known in the art. As used herein, "whitening" refers to a change in visual appearance of a tooth, preferably such that the tooth has a brighter shade. Increase in whiteness of a dental surface can be observed visually, for example with the aid of color comparison charts or gauges, or measured by colorimetry, using any suitable instrument such as a Minolta Chromameter, e.g., model CR-400 (Minolta Corp., Ramsey, N.J.). The instrument can be programmed, for example, to measure Hunter Lab values or L*a*b* values according to the standard established by the International Committee of Illumination (CIE). The L*a*b* system provides a numerical representation of three-dimensional color space where L* represents a lightness axis, a* represents a red-green axis and b* represents a yellowblue axis. The L* and b* axes are typically of greatest applicability to measurement of tooth whiteness.

In certain embodiments, an increase in whiteness can be computed from differences in L*, a* and b* values before and after treatment, or between untreated and treated surfaces. A useful parameter is $\Delta E^*$, calculated as the square root of the sum of the squares of differences in L*, a* and b* values, using the formula:

$$\Delta E^* = [(\Delta L^*)^2 + (\Delta a^*)^2 + (\Delta b^*)^2]^{1/2}.$$

A higher value of $\Delta E^*$ indicates greater increase in whiteness. In various embodiments, the tooth whitening compositions disclosed herein can effect a $\Delta E^*$ of at least about 1, such as at least about 3, at least about 4, or at least about 5.

In certain exemplary embodiments, whiteness may be measured by a $\Delta W$. In certain embodiments, whiteness $W = 100 - \sqrt{((100-L^{*2}) + a^{*2} + b^{*2})}$, wherein L*, a*, and b* are as defined above. Accordingly, $\Delta W = W_O - W_T$, wherein $W_O =$ initial whiteness and $W_T =$ whiteness after treatment.

Further disclosed herein are methods of making a tooth whitening composition as disclosed herein. The tooth whitening compositions disclosed herein may, in certain embodiments, be prepared by adding and mixing the ingredients of the composition in a suitable vessel, such as a stainless steel tank provided with a mixer. In the preparation of the tooth whitening composition, the ingredients may be added to the mixer in the following order: hydrophobic polymer carrier, peroxide compounds, adhesion enhancing agents, optional peroxide activation agents, and any desired flavoring agents and/or sweeteners. The ingredients are then mixed to form a homogeneous dispersion/solution.

The tooth whitening composition disclosed herein may be prepared in the form of a flowable viscous liquid dispersion, such as a gel, comprising at least one whitening agent and is applied as such to the user's teeth as by painting the teeth with a soft applicator brush. Application by the user leaves a coating of the composition on the teeth. Contact with saliva causes the slow release of $H_2O_2$ from the hydrophobic material matrix to the applied tooth site from the composition, providing prolonged whitening treatment of the tooth sites.

## EXAMPLES

### Comparative Example 1

Four tooth whitening compositions were prepared and applied in vitro to bovine teeth for 8 treatments. After each treatment, whiteness was measured and the ΔW calculated as described above. The four compositions prepared contained the following agents: (1) 4.5% hydrogen peroxide (complexed with PVP); (2) 4.5% hydrogen peroxide (complexed with PVP) and 1.0% sodium peroxy disulfate (PDS); (3) 4.5% hydrogen peroxide (complexed with PVP) and 1.0% sodium monoperoxysulfate (oxone) (MPS); and (4) 4.5% hydrogen peroxide (complexed with PVP) and 1.0% sodium pyrophosphate (TKPP). The results are shown below in Table 1.

As shown in Table 1, none of PDS, MPS, or TKPP enhanced whitening when combined with hydrogen peroxide. To the contrary, after 8 treatments, the addition of PDS, MPS, or TKPP appeared to decrease the whitening efficacy of 4.5% hydrogen peroxide.

Table 1 - Change in Whitening for In-Vitro Bovine Teeth Bleaching of Compositions Comprising 4.5% Hydrogen Peroxide and Other Agents

| Treatment | 4.5% HP | 4.5% HP + 1.0% PDS | 4.5% HP + 1.0% MPS | 4.5% HP + 1.0% TKPP |
|---|---|---|---|---|
| 0 | 0.00 | 0.00 | 0.00 | 0.00 |
| 2 | 2.30 | 2.33 | 2.25 | 0.90 |
| 4 | 3.84 | 3.43 | 3.65 | 1.93 |
| 6 | 5.02 | 4.63 | 4.33 | 2.97 |
| 8 | 5.66 | 5.10 | 4.36 | 3. 8 1 |

Attempts to introduce alkaline ingredients such as calcium oxide and similar compounds also failed. Peroxide instability and product separation were observed. While not wishing to be bound by theory, it is believed that the hydrophobic nature of the compositions may make it more challenging to release whitening agents within the peroxide complex matrix to provide whitening efficacy beyond the initial formulation (i.e., 4.5% hydrogen peroxide).

### Comparative Example 2

Two compositions were prepared - one comprising 4.5% hydrogen peroxide (complexed with PVP) and one comprising 6.0% hydrogen peroxide (Complexed with PVP). The two compositions were then applied in vitro to bovine teeth for 8 treatments. After each treatment, whiteness was measured and the ΔW calculated as discussed above. The results are shown below in Table 2.

As shown in Table 2, increasing the hydrogen peroxide percentage to 6.0% failed to significantly increase whitening efficacy.

Table 2 - Change in Whitening for In-Vitro Bovine Teeth Bleaching of Compositions Comprising 4.5% and 6.0% Hydrogen Peroxide

| Treatment | 4.5% HP | 6.0% HP |
|---|---|---|
| 0 | 0 | 0 |
| 2 | 2.02 | 2.37 |
| 4 | 3.58 | 3.53 |
| 6 | 4.70 | 5.51 |
| 8 | 5.44 | 5.77 |
| 10 | 6.61 | 6.27 |

### Example 3

A first composition was prepared comprising 4.5% hydrogen peroxide and no additional peroxide source, and a second composition was prepared comprising 4.5% hydrogen peroxide and 2% sodium percarbonate. The two compositions are shown below in Table 3. The two compositions were then applied in vitro for 5 daily treatments. After each treatment, whiteness was measured and the ΔW calculated. The results are shown below in Table 4. As shown in Table 4, the addition of 2.0% sodium percarbonate to 4.5% hydrogen peroxide increased whitening efficacy of the composition.

Table 3 - Composition A (4.5% HP) and Composition B (4.5% HP and 2% Sodium Percarbonate)

| Ingredient | Comp A - 4.5% HP (% by weight) | Comp B - 4.5% HP + 2% Sodium Percarbonate (% by weight) |
|---|---|---|
| Dimethicone | 32% | 31% |
| Mineral oil | 28.6% | 27.5% |
| PVP-Hydrogen Peroxide complex | 25% (20.5% PVP/4.5% HP) | 25% (20.5% PVP/4.5% HP) |
| Trimethylsiloxysilicate | 12% | 12% |
| Sodium percarbonate peroxi de | -- | 2% |
| Polyethylene | 1.5% | 1.5% |
| Flavorants and sweeteners | QS | QS |

Table 4 - Change in Whitening for Teeth Bleaching of Compositions Comprising 4.5% Hydrogen and 4.5% Plus 2.0% Sodium Percarbonate

| Treatment | 4.5% HP | 4.5% HP + 2.0% Sodium Percarbonate |
|---|---|---|
| 1 | 3.7 | 6.0 |
| 2 | 5.5 | 8.6 |
| 3 | 6.7 | 10.3 |
| 4 | 7.9 | 10.9 |
| 5 | 9.1 | 11.8 |

**Claims**

1. A tooth whitening composition comprising:

   (a) a peroxide complex comprising hydrogen peroxide and an N-vinyl heterocyclic polymer;
   (b) from 0.1% to 6%, by weight, sodium percarbonate; and
   (c) at least one hydrophobic polymer carrier,

   wherein the hydrogen peroxide of the peroxide complex is present in the tooth whitening composition in an amount ranging from 3% to 5% by weight.

2. The tooth whitening composition according to claim 1, wherein the hydrogen peroxide of the peroxide complex is present in the tooth whitening composition in an amount of 4.5% by weight.

3. The tooth whitening composition according to claim 1, wherein the sodium percarbonate is present in an amount of 2% by weight.

4. The tooth whitening composition according to claim 1, wherein the at least one hydrophobic carrier is chosen from polysiloxane polymers.

5. The tooth whitening composition according to claim 1, wherein the at least one hydrophobic carrier is chosen from dimethicone, trimethylsiloxysilicate, and cyclomethicone.

6. The tooth whitening composition according to claim 1, wherein the tooth whitening composition has a viscosity ranging from 10,000 cps to 900,000 cps, measured on a Brookfield Viscometer at 25 °C.

7. A method for whitening a surface of a tooth comprising:

contacting the surface of a tooth with a tooth whitening composition for a duration of time sufficient to effect whitening of the surface of the tooth,
wherein the tooth whitening composition comprises a peroxide complex comprising hydrogen peroxide and an N-vinyl heterocyclic polymer, from 0.1% to 6%, by weight, sodium percarbonate, and at least one hydrophobic polymer carrier, and
wherein the hydrogen peroxide of the peroxide complex is present in the tooth whitening composition in an amount ranging from 3% to 5% by weight.

8. The method according to claim 7, wherein the duration of time ranges from 1 minute to 45 minutes.

9. The method according to claim 7, wherein the hydrogen peroxide of the peroxide complex is present in the tooth whitening composition in an amount of 4.5% by weight.

10. The method according to claim 7, wherein the sodium percarbonate is present in an amount of 2% by weight.

11. A method for making a tooth whitening composition, comprising:

adding a peroxide complex comprising hydrogen peroxide and an N-vinyl heterocyclic polymer and from 0.1% to 6%, by weight, sodium percarbonate to at least one hydrophobic polymer carrier; and
mixing the peroxide complex, the sodium percarbonate, and the at least one hydrophobic polymer carrier to form a homogeneous dispersion,
wherein the hydrogen peroxide is present in the tooth whitening composition in an amount ranging from 3% to 5% by weight.

12. The method according to claim 11, wherein the hydrogen peroxide of the peroxide complex is present in the tooth whitening composition in an amount of 4.5% by weight.

13. The method according to claim 11, wherein the sodium percarbonate is present in an amount of 2% by weight.

14. The method according to claim 11, further comprising adding at least one flavoring agent.

**Patentansprüche**

1. Zahnaufhellungszusammensetzung, umfassend:

(a) einen Peroxidkomplex umfassend Wasserstoffperoxid und ein N-Vinyl-Heterocycluspolymer;
(b) 0,1 Gew.-% bis 6 Gew.-% Natriumpercarbonat; und
(c) mindestens einen hydrophoben Polymerträger,

wobei das Wasserstoffperoxid des Peroxidkomplexes in der Zahnaufhellungszusammensetzung in einer Menge im Bereich von 3 Gew.-% bis 5 Gew.-% vorhanden ist.

2. Zahnaufhellungszusammensetzung nach Anspruch 1, wobei das Wasserstoffperoxid in den Peroxidkomplexes in der Zahnaufhellungszusammensetzung in einer Menge von 4,5 Gew.-% vorliegt.

3. Zahnaufhellungszusammensetzung nach Anspruch 1, wobei das Natriumpercarbonat in einer Menge von 2 Gew.-% vorliegt.

4. Zahnaufhellungszusammensetzung nach Anspruch 1, wobei der mindestens eine hydrophobe Träger ausgewählt ist aus Polysiloxanpolymeren.

5. Zahnaufhellungszusammensetzung nach Anspruch 1, wobei der mindestens eine hydrophobe Träger ausgewählt ist aus Dimethicon, Trimethylsiloxysilicat und Cyclomethicon.

**6.** Zahnaufhellungszusammensetzung nach Anspruch 1, wobei die Zahnaufhellungszusammensetzung eine Viskosität in einem Bereich von 10.000 cps bis 900.000 cps hat, gemessen auf einem Brookfield-Viskosimeter bei 25 °C.

**7.** Verfahren zur Aufhellung einer Oberfläche eines Zahns, umfassend:

Inkontaktbringen der Oberfläche eines Zahns mit einer Zahnaufhellungszusammensetzung über einen Zeitraum für eine ausreichende Zeit um eine Aufhellung der Oberfläche des Zahns zu erreichen, wobei die Zahnaufhellungszusammensetzung einen Peroxidkomplex umfasst, umfassend Wasserstoffperoxid und ein N-Vinyl-Heterocycluspolymer, von 0,1 Gew.-% bis 6 Gew.-% Natriumpercarbonat und mindestens eine hydrophobe polymere Träger, und wobei das Wasserstoffperoxid in dem Peroxidkomplexes in der Zahnaufhellungszusammensetzung in einer Menge im Bereich von 3 Gew.-% bis 5 Gew.-% vorliegt.

**8.** Verfahren nach Anspruch 7, wobei die Zeitdauer in einem Bereich von 1 Minute bis 45 Minuten liegt.

**9.** Verfahren nach Anspruch 7, wobei das Wasserstoffperoxid des Peroxidkomplexes in der Zahnweißzusammensetzung in einer Menge von 4,5 Gew.-% vorliegt.

**10.** Verfahren nach Anspruch 7, wobei das Natriumpercarbonat in einer Menge von 2 Gew.-% vorliegt.

**11.** Verfahren zur Herstellung einer Zahnaufhellungszusammensetzung, umfassend:

Zugabe eines Peroxidkomplexes umfassend Wasserstoffperoxid und ein N-Vinyl-Heterocycluspolymer und 0,1 Gew.-% bis 6 Gew.-% Natriumpercarbonat zu mindestens einem hydrophoben Polymerträger; und Vermischen des Peroxidkomplexes des Natriumpercarbonats und des mindestens einen hydrophoben Polymerträgers, um eine homogene Dispersion zu bilden, wobei das Wasserstoffperoxid in der Zahnaufhellungszusammensetzung in einer Menge in einem Bereich von 3 Gew.-% bis 5 Gew.-% vorliegt.

**12.** Verfahren nach Anspruch 11, wobei das Wasserstoffperoxid des Peroxidkomplexes in der Zahnweißzusammensetzung in einer Menge von 4,5 Gew.-% vorliegt.

**13.** Verfahren nach Anspruch 11, wobei das Natriumpercarbonat in einer Menge von 2 Gew.-% vorliegt.

**14.** Verfahren nach Anspruch 11, weiterhin umfassend Zugabe von mindestens einem Geschmacksmittel.

**Revendications**

**1.** Composition de blanchiment des dents comprenant :

(a) un complexe de peroxyde comprenant du peroxyde d'hydrogène et un polymère hétérocyclique N-vinylique ; (b) de 0,1 % à 6 %, en poids, de percarbonate de sodium ; et (c) au moins un support polymère hydrophobe,

dans laquelle le peroxyde d'hydrogène du complexe de peroxyde est présent dans la composition de blanchiment des dents en une quantité allant de 3 % à 5 % en poids.

**2.** Composition de blanchiment des dents selon la revendication 1, dans laquelle le peroxyde d'hydrogène du complexe de peroxyde est présent dans la composition de blanchiment des dents en une quantité de 4,5 % en poids.

**3.** Composition de blanchiment des dents selon la revendication 1, dans laquelle le percarbonate de sodium est présent en une quantité de 2 % en poids.

**4.** Composition de blanchiment des dents selon la revendication 1, dans laquelle l'au moins un support hydrophobe est choisi parmi les polymères polysiloxanes.

**5.** Composition de blanchiment des dents selon la revendication 1, dans laquelle l'au moins un support hydrophobe est choisi parmi la diméthicone, le triméthylsiloxysilicate et la cyclométhicone.

6. Composition de blanchiment des dents selon la revendication 1, dans laquelle la composition de blanchiment des dents a une viscosité allant de 10 000 cps à 900 000 cps, mesurée sur un viscosimètre Brookfield à 25 °C.

7. Procédé de blanchiment d'une surface d'une dent comprenant :

la mise en contact de la surface d'une dent avec une composition de blanchiment des dents pendant une durée suffisante pour effectuer un blanchiment de la surface de la dent,
dans lequel la composition de blanchiment des dents comprend un complexe de peroxyde comprenant du peroxyde d'hydrogène et un polymère hétérocyclique N-vinylique, de 0,1 % à 6 %, en poids, de percarbonate de sodium et au moins un support polymère hydrophobe, et
dans lequel le peroxyde d'hydrogène du complexe de peroxyde est présent dans la composition de blanchiment des dents en une quantité allant de 3 % à 5 % en poids.

8. Procédé selon la revendication 7, dans lequel la durée varie de 1 minute à 45 minutes.

9. Procédé selon la revendication 7, dans lequel le peroxyde d'hydrogène du complexe peroxyde est présent dans la composition de blanchiment des dents en une quantité de 4,5 % en poids.

10. Procédé selon la revendication 7, dans lequel le percarbonate de sodium est présent en une quantité de 2 % en poids.

11. Procédé de fabrication d'une composition de blanchiment des dents, comprenant :

l'ajout d'un complexe de peroxyde comprenant du peroxyde d'hydrogène et un polymère hétérocyclique N-vinylique et de 0,1 % à 6 %, en poids, de percarbonate de sodium à au moins un support polymère hydrophobe ; et
le mélange du complexe de peroxyde, du percarbonate de sodium et de l'au moins un support polymère hydrophobe pour former une dispersion homogène,
dans lequel le peroxyde d'hydrogène est présent dans la composition de blanchiment des dents en une quantité allant de 3 % à 5 % en poids.

12. Procédé selon la revendication 11, dans lequel le peroxyde d'hydrogène du complexe de peroxyde est présent dans la composition de blanchiment des dents en une quantité de 4,5 % en poids.

13. Procédé selon la revendication 11, dans lequel le percarbonate de sodium est présent en une quantité de 2 % en poids.

14. Procédé selon la revendication 11, comprenant en outre l'ajout d'au moins un agent aromatisant.

**REFERENCES CITED IN THE DESCRIPTION**

**Patent documents cited in the description**

- US 2005036956 A1
- US 2006045854 A1
- US 20150305843 A1